(19) Europäisches Patentamt
European Patent Office
Office européen des brevets

(11) Veröffentlichungsnummer: **0 327 792 B1**

(12) # EUROPÄISCHE PATENTSCHRIFT

(45) Veröffentlichungstag der Patentschrift: **22.12.93**

(51) Int. Cl.[5]: **C07D 209/46**, C07C 229/34

(21) Anmeldenummer: **89100028.3**

(22) Anmeldetag: **03.01.89**

(54) **3-(4-Amino-2-hydroxyphenyl)-1-oxoisoindolenine, Verfahren zu deren Herstellung und deren Verseifung zu 2-(4-Amino-2-hydroxybenzoyl)-benzoesäuren.**

(30) Priorität: **12.01.88 DE 3800577**

(43) Veröffentlichungstag der Anmeldung:
**16.08.89 Patentblatt 89/33**

(45) Bekanntmachung des Hinweises auf die Patenterteilung:
**22.12.93 Patentblatt 93/51**

(84) Benannte Vertragsstaaten:
**CH DE FR GB IT LI**

(56) Entgegenhaltungen:
**EP-A- 0 176 161**
**DE-A- 1 445 489**

**JOURNAL OF THE AMERICAN CHEMICAL SOCIETY, (C), 1969; R.K. BARTLETT et al.: "The reactions of phenols with phthalodinitrile and 3-amino-1H-isoindoles", Seiten 129-133**

**ANNALEN DER CHEMIE, Band 594, 1955; G. WITTIG et al.: "Ueber Ringerweiterung und Ringverengerung auf der Basis von Ylidisomerisationen", Seiten 89-118**

(73) Patentinhaber: **BASF Aktiengesellschaft**
**Carl-Bosch-Strasse 38**
**D-67063 Ludwigshafen(DE)**

(72) Erfinder: **Kranz, Joachim, Dr.**
**Rheinrugenstrasse 22**
**D-6700 Ludwigshafen(DE)**
Erfinder: **Landmann, Bernd, Dr.**
**Budapester Strasse 45**
**D-6700 Ludwigshafen(DE)**
Erfinder: **Mayer, Udo, Dr.**
**Max-Slevogt-Strasse 27**
**D-6710 Frankenthal(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.6/3.3.1)

**Beschreibung**

2-(4-Amino-2-hydroxybenzoyl)-benzoesäuren (IV) sind wichtige Zwischenprodukte bei der Herstellung von Fluoranen und Rhodaminen. Die Säuren (IV) werden nach dem Stand der Technik durch Umsetzen von 3-Aminophenolen mit gegebenenfalls substituierten Phthalsäureanhydriden hergestellt (DE-A-85 931; DE-A-23 22 131 und 24 44 297; US-A-45 15 971; DE-A-34 15 331; EP-A-176 161; JP-A-70 350/1987). Bei dieser Reaktion werden Rhodamine als Nebenprodukte gebildet. Die gewünschten Benzoylbenzoesäuren (IV) entstehen oft nur in niedrigen Ausbeuten. Bestimmte Benzoylbenzoesäuren sind auf diesem Weg überhaupt nicht darstellbar.

Aufgabe der vorliegenden Erfindung war es, ein technisch gut durchführbares Verfahren zu Herstellung von Benzoylbenzoesäuren aufzufinden, nach dem die gewünschten Benzoylbenzoesäuren in guter bis hoher Ausbeute und hoher Reinheit erhalten werden können. Dabei wurde gefunden, daß sich Benzoylbenzoesäuren (IV) gut aus den neuen Isoindolenin-Verbindungen (I) herstellen lassen.

Dementsprechend betrifft die Erfindung neue Isoindolenine der allgemeinen Formel (I)

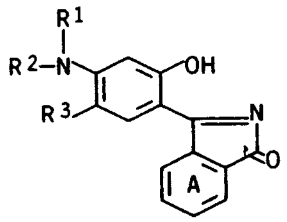

(I),

in der

$R^1$ für Wasserstoff, für gegebenenfalls durch Chlor, Hydroxy, Phenyl oder Cyan substituiertes $C_1$-$C_{12}$-Alkyl, $C_5$-$C_8$-Cycloalkyl oder für gegebenenfalls durch Chlor oder Methyl substituiertes Phenyl,

$R^2$ für Wasserstoff, gegebenenfalls durch Chlor, Hydroxy, Phenyl oder Cyan substituiertes $C_1$-$C_6$-Alkyl oder

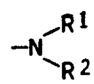

für einen Morpholino-, Pyrrolidino- oder Piperidinorest und

$R^3$ für Wasserstoff oder Methyl stehen und der Benzolring A gegebenenfalls durch 1 bis 4 Chlor, 1 oder 2 $C_1$-$C_4$-Alkyl oder eine Nitrogruppe substituiert ist.

Weiterhin betrifft die Erfindung ein Verfahren zur Herstellung von Isoindoleninen der allgemeinen Formel I, das dadurch gekennzeichnet ist, daß man Aminophenole der allgemeinen Formel (II)

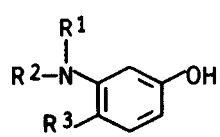

(II),

in der

$R^1$ für Wasserstoff, für gegebenenfalls durch Chlor, Hydroxy, Phenyl oder Cyan substituiertes $C_1$-$C_{12}$-Alkyl, $C_5$-$C_8$-Cycloalkyl oder für gegebenenfalls durch Chlor oder Methyl substituiertes Phenyl,

$R^2$ für Wasserstoff, gegebenenfalls durch Chlor, Hydroxy, Phenyl oder Cyan substituiertes $C_1$-$C_6$-Alkyl oder

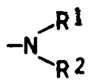

für einen Morpholino-, Pyrrolidino- oder Piperidinorest und

$R^3$ für Wasserstoff oder Methyl stehen,

mit 3-Amino-1-oxo-isoindoleninen der allgemeinen Formel (III)

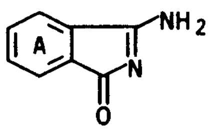

(III),

in der der Benzolring A gegebenenfalls durch 1 bis 4 Chlor, 1 oder 2 $C_1$-$C_4$-Alkyl oder eine Nitrogruppe substituiert ist, in Gegenwart einer Säure kondensiert.

Die erfindungsgemäßen Isoindolenine I werden vorteilhafterweise durch Kondensation des 3-Aminophenols (II) mit dem 3-Amino-1-oxo-isoindolenin (III) in einem organischen Lösungsmittel in Gegenwart eines Äquivalents einer Säure bei 60 bis 140 °C hergestellt. Als Lösungsmittel kommen z.B. Dimethylformamid, Toluol, Xylol, Chlorbenzol, Dichlorbenzol und geradkettige oder verzweigte Alkanole in Betracht. Bevorzugte Lösungsmittel sind Dimethylformamid und Isobutanol. Als Säuren können z.B. Salzsäure, Schwefelsäure, Phosphorsäure oder p-Toluolsulfonsäure verwendet werden. Vorzugsweise verwendet man zur Kondensation das Isoindolenin-Hydrochlorid der Formel (IIIa)

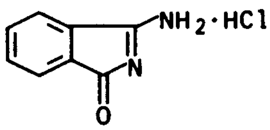

(IIIa).

Die neuen Isoindolenine (I) werden in hoher Ausbeute erhalten und lassen sich in hoher Ausbeute in die entsprechenden Benzoylbenzoesäuren der allgemeinen Formel (IV)

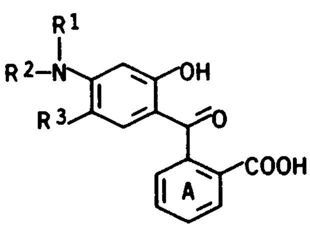

(IV),

in der $R^1$, $R^2$, $R^3$ und A die oben angegebene Bedeutung haben, überführen.

Dementsprechend betrifft die Erfindung außerdem ein Verfahren zur Herstellung von Benzoylbenzoesäuren (IV), bei dem Verbindungen der allgemeinen Formel (I) verseift werden. Die Verseifung kann sauer oder vorzugsweise unter alkalischen Bedingungen erfolgen. Die erhaltenen Benzoylbenzoesäuren (IV) sind sehr rein und nicht durch Rhodamine verunreinigt. Einige Benzoylbenzoesäuren sind nach den Verfahren des Standes der Technik (Kondensation des Aminophenols mit Phthalsäureanhydrid) entweder nicht oder nur in geringer Ausbeute darstellbar.

Die Verseifung erfolgt in der Regel durch Erwärmen von (I) in verdünnter Säure oder vorzugsweise in wäßriger Alkalimetallhydroxidlösung, wobei im letzteren Fall die Benzoylbenzoesäure in Lösung geht.

Als Säure verwendet man 5 bis 30 gew.%ige Säuren wie Schwefelsäure oder Salzsäure.

Als Basen verwendet man vorzugsweise 5 bis 40 gew.%ige wäßrige Lösungen von Alkali- und Erdalkalimetallhydroxid, insbesondere Natron- oder Kalilauge. Die Säure (IV) wird durch Ansäuern aus der alkalischen Lösung gefällt und in üblicher Weise isoliert.

Die Erfindung soll durch die folgenden Beispiele zusätzlich erläutert werden. Die in den Beispielen genannten Teile und Prozentangaben beziehen sich auf das Gewicht.

Beispiel 1

a) In eine Lösung von 151 Teilen 3-Ethylamino-4-methylphenol in 240 Teilen Dimethylformamid werden bei 120°C portionsweise 183 Teile 3-Amino-1-oxo-isoindolenin-hydrochlorid eingetragen. Die Mischung wird eine Stunde lang auf 120 °C erhitzt. Nach dem Erkalten wird der Feststoff abgesaugt, mit 200 Teilen Ethanol gewaschen und bei 60°C im Vakuum getrocknet. Man erhält 263 Teile 3-(4'-Ethylamino-2'-hydroxy-5'-methylphenyl)-1-oxo-isoindolenin als braune Kristalle vom Schmelzpunkt 258 bis 260°C.

| $C_{17}H_{16}N_2O_2$ MM 280,33 | | | | |
|---|---|---|---|---|
| | C | H | N | O |
| ber.: | 72,84 | 5,75 | 9,99 | 11,41 |
| gef.: | 72,6 | 5,9 | 10,2 | 11,4 |

b) 28 Teile 3-(4'-Ethylamino-2'-hydroxy-5'-methylphenyl)-1-oxo-isoindolenin werden in 118 Teilen 20 %iger Kalilauge 5 Stunden lang unter Rückfluß erhitzt. Die Lösung wird filtriert und das Filtrat nach dem Abkühlen mit konzentrierter Salzsäure auf pH 4,5 gestellt. Der ausgefallene Feststoff wird abgesaugt, mit Wasser gewaschen und im Vakuum bei 60°C getrocknet. Man erhält 23,5 Teile 2-(4'-Ethylamino-2'-hydroxy-5'-methylbenzoyl)-benzoesäure als gelblichen Feststoff vom Schmelzpunkt 200 bis 202°C.

| $C_{17}H_{17}NO_4$ MM 299,33 | | | | |
|---|---|---|---|---|
| | C | H | N | O |
| ber.: | 68,22 | 5,72 | 4,68 | 21,38 |
| gef.: | 68,1 | 5,7 | 4,7 | 21,2 |

Beispiel 2

a) In eine Lösung von 17,9 Teilen 3-Morpholinophenol in 24 Teilen Isobutanol werden bei 100°C portionsweise 18,3 Teile 3-Amino-1-oxo-isoindolenin-hydrochlorid eingetragen. Die Mischung wird 5 Stunden lang auf 100°C erhitzt. Nach dem Erkalten wird der Feststoff abgesaugt, mit Isobutanol gewaschen und im Vakuum bei 60°C getrocknet. Man erhält 24,1 Teile 3-(4'-Morpholino-2'-hydroxyphenyl)-1-oxo-isoindolenin als rotbraunen Feststoff. Eine Probe wird aus Dimethylformamid umkristallisiert: rotbraune Nadeln vom Schmelzpunkt 290 bis 292°C.

| $C_{18}H_{16}N_2O_2$ MM 308,34 | | | | |
|---|---|---|---|---|
| | C | H | N | O |
| ber.: | 70,12 | 5,23 | 9,09 | 15,57 |
| gef.: | 69,9 | 5,4 | 9,2 | 15,5 |

b) 15,4 Teile 3-(4'-Morpholino-2'-hydroxyphenyl)-1-oxo-isoindolenin werden in 60 Teilen 20 %iger Natronlauge 1 Stunde lang unter Rückfluß erhitzt. Die Lösung wird filtriert und das Filtrat nach dem Abkühlen mit konzentrierter Salzsäure auf pH 4,5 gestellt. Der ausgefallene Feststoff wird abgesaugt, mit Wasser gewaschen und im Vakuum bei 60°C getrocknet. Man erhält 10,7 Teile 2-(4'-Morpholino-2'-hydroxybenzoyl)-benzoesäure als gelblichen Feststoff vom Schmelzpunkt 184 bis 187°C.

| $C_{18}H_{17}NO_5$ MM 327,34 | | | | |
|---|---|---|---|---|
| | C | H | N | O |
| ber.: | 66,05 | 5,23 | 4,28 | 24,44 |
| gef.: | 65,9 | 5,0 | 4,0 | 24,4 |

Analog Beispiel 2a) und 2b) werden die substituierten 1-Oxo-isoindolenine (VI) und Benzoylbenzoesäuren (VII) hergestellt.

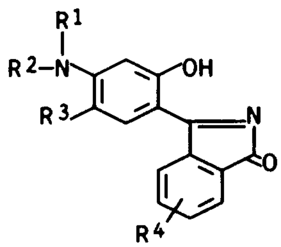

(VI)

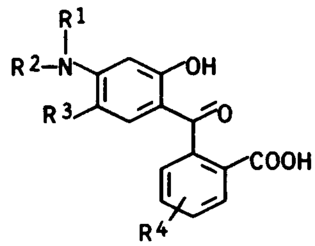

(VII)

Die Verbindungen sind in Tabelle 1 durch ihre Schmelzpunkte charakterisiert.

Tabelle 1

| Nr. | $R^1$ | $R^2$ | $R^3$ | $R^4$ | (VI) Schmp. [°C] | (VII) Schmp. [°C] |
|---|---|---|---|---|---|---|
| 3 | 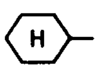 | $-H$ | $-H$ | $-H$ | 242-246 | 126 - 128 |
| 4 | 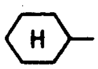 | $-CH_3$ | $-H$ | $-H$ | 227-230 (Zers.) | 125-130 |
| 5 | $-CH_3$ | $-CH_3$ | $-H$ | $-H$ | 250-253 | 137-140 |
| 6 | $-C_2H_5$ | $-C_2H_5$ | $-H$ | $-H$ | 250-253 | 214-217 |

Tabelle 1 (Fortsetzung)

| Nr. | $R^1$ | $R^2$ | $R^3$ | $R^4$ | (VI) Schmp. [°C] | (VII) Schmp. [°C] |
|---|---|---|---|---|---|---|
| 7 | $-C_2H_4OH$ | $-CH_3$ | $-H$ | $-H$ | 216-218 | 102-104 |
| 8 | $-(CH_2)_4-$ | | $-H$ | $-H$ | 238-242 | 159-162 |
| 9 | $-CH_3$ | $-CH_3$ | $-CH_3$ | $-H$ | | 182-185 |
| 10 | $-C_2H_5$ | $-CH_3$ | $-CH_3$ | $-H$ | | 152-154 |

Außerdem wurden auf dem gleichen Wege die in der folgenden Tabelle aufgeführten substituierten 1-Oxo-isoindolenine (VI) und Benzoylbenzoesäuren (VII) hergestellt.

Tabelle 2

| Nr. | $R^1$ | $R^2$ | $R^3$ | $R^4$ |
|---|---|---|---|---|
| 11 | 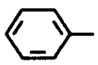 | $-CH_3$ | $-H$ | $-H$ |
| 12 | 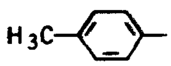 | $-CH_3$ | $-H$ | $-H$ |
| 13 | 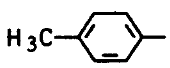 | $C_2H_5$ | $-H$ | $-H$ |
| 14 | 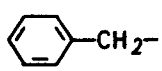 | H | $-CH_3$ | $-H$ |
| 15 | $-(CH_2)_4-$ | | $-H$ | $-C(CH_3)_3$ |
| 16 | $-(CH_2)_2-O-(CH_2)_2-$ | | $-H$ | $-C(CH_3)_3$ |
| 17 | $-(CH_2)_5-$ | | $-H$ | $-C(CH_3)_3$ |
| 18 | $-CH_3$ | $-CH_3$ | $-H$ | $-C(CH_3)_3$ |
| 19 | $-C_2H_5$ | $-C_2H_5$ | $-H$ | $-C(CH_3)_3$ |
| 20 | $-C_4H_9$ | $-C_4H_9$ | $-H$ | $-H$ |
| 21 | 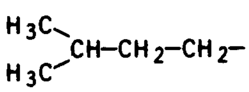 | $-C_2H_5$ | $-H$ | $-H$ |

**Patentansprüche**

**1.** Isoindolenine der allgemeinen Formel (I)

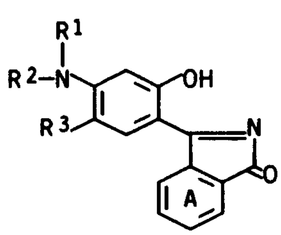

(I),

in der

$R^1$ für Wasserstoff, für gegebenenfalls durch Chlor, Hydroxy, Phenyl oder Cyan substituiertes $C_1$-$C_{12}$-Alkyl, $C_5$-$C_8$-Cycloalkyl oder für gegebenenfalls durch Chlor oder Methyl substituiertes Phenyl,

$R^2$ für Wasserstoff, gegebenenfalls durch Chlor, Hydroxy, Phenyl oder Cyan substituiertes $C_1$-$C_6$-Alkyl oder

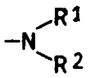

für einen Morpholino-, Pyrrolidino- oder Piperidinorest und

$R^3$ für Wasserstoff oder Methyl stehen und der Benzolring A gegebenenfalls durch 1 bis 4 Chlor, 1 oder 2 $C_1$-$C_4$-Alkyl oder eine Nitrogruppe substituiert ist.

2. Isoindolenine nach Anspruch 1, dadurch gekennzeichnet, daß $R^1$ für $C_1$-$C_6$-Alkyl, Benzyl, Phenyl oder Methylphenyl steht.

3. Isoindolenine nach Anspruch 1, dadurch gekennzeichnet, daß $R^1$ für Methyl oder Ethyl steht.

4. Isoindolenine nach Anspruch 1, 2 oder 3, dadurch gekennzeichnet daß $R^2$ für Wasserstoff, Methyl oder Ethyl steht.

5. Isoindolenine nach Anspruch 1, 2, 3 oder 4, dadurch gekennzeichnet daß $R^3$ für Methyl steht.

6. Isoindolenine nach Anspruch 1, dadurch gekennzeichnet, daß

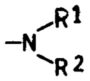

für einen Morpholino-, Pyrrolidino- oder Piperidinorest und $R^3$ für Wasserstoff stehen.

7. Isoindolenine nach Anspruch 1, 2, 3, 4, 5 oder 6, dadurch gekennzeichnet, daß der Benzolring A unsubstituiert oder durch t-Butyl substituiert ist.

8. Verfahren zur Herstellung von Isoindoleninen gemäß Anspruch 1, dadurch gekennzeichnet, daß man 3-Aminophenole der allgemeinen Formel (II)

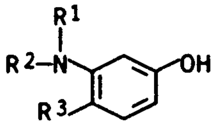

(II),

in der $R^1$, $R^2$ und $R^3$ die in Anspruch 1 angegebene Bedeutung haben, mit 3-Amino-1-oxo-isoindoleninen der allgemeinen Formel (III)

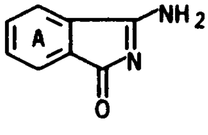

(III),

in der der Benzolring A gegebenenfalls durch 1 bis 4 Chlor, 1 oder 2 $C_1$-$C_4$-Alkyl oder eine Nitrogruppe substituiert ist, in Gegenwart einer Säure kondensiert.

9. Verfahren nach Anspruch 8, dadurch gekennzeichnet, daß die Kondensation in einem organischen Lösungsmittel in Gegenwart eines Äquivalents einer Säure bei 60 bis 140 °C erfolgt.

10. Verfahren zur Herstellung von 2-(4-Amino-2-hydroxybenzoyl)-benzoesäuren der allgemeinen Formel (IV)

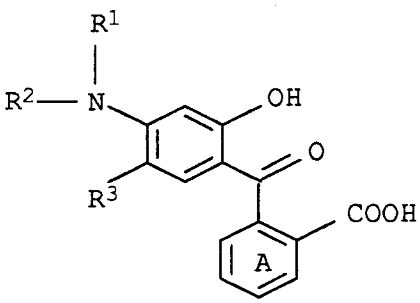

(IV),

in der

$R^1$ für Wasserstoff, für gegebenenfalls durch Chlor, Hydroxy, Phenyl oder Cyan substituiertes $C_1$-$C_{12}$-Alkyl,$C_5$-$C_8$-Cycloalkyl oder für gegebenenfalls durch Chlor oder Methyl substituiertes Phenyl,

$R^2$ für Wasserstoff, gegebenenfalls durch Chlor, Hydroxy, Phenyl oder Cyan substituiertes $C_1$-$C_6$-Alkyl oder

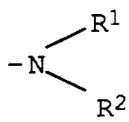

für einen Morpholino-, Pyrrolidino- oder Piperidinorest und

$R^3$ für Wasserstoff oder Methyl stehen und der Benzolring A gegebenenfalls durch 1 bis 4 Chlor, 1 oder 2 $C_1$-$C_4$-Alkyl oder eine Nitrogruppe substituiert ist,

dadurch gekennzeichnet, daß man

a) 3-Aminophenole der allgemeinen Formel (II)

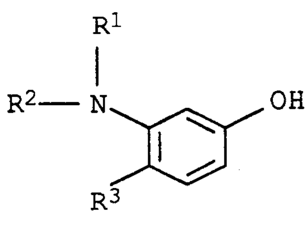

(II)

mit 3-Amino-1-oxo-isoindoleninen der allgemeinen Formel (III)

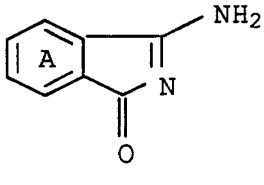

(III)

in Gegenwart einer Säure zu 3-(4-Amino-2-hydroxyphenyl)-1-oxo-isoindoleninen der allgemeinen Formel (I)

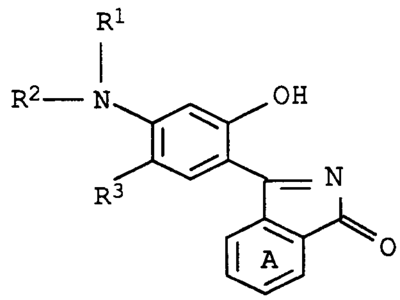

(I),

kondensiert und

b) die erhaltenen 3-(4-Amino-2-hydroxyphenyl)-1-oxo-isoindolenine (I) verseift.

**Claims**

1. An isoindolenine of the general formula (I)

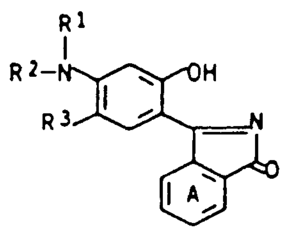

(I)

where

$R^1$ is hydrogen, unsubstituted or chlorine-, hydroxyl-, phenyl- or cyano-substituted $C_1$-$C_{12}$-alkyl or $C_5$-$C_8$-cycloalkyl or unsubstituted or chlorine- or methyl-substituted phenyl,

$R^2$ is hydrogen or unsubstituted or chlorine-, hydroxyl-, phenyl- or cyano-substituted $C_1$-$C_6$-alkyl, or is morpholino, pyrrolidino or piperidino, and

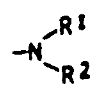

$R^3$ is hydrogen or methyl and the benzene ring A may be substituted by from 1 to 4 chlorines, 1 or 2 $C_1$-$C_4$-alkyls or 1 nitro.

2. An isoindolenine as claimed in claim 1, wherein $R^1$ is $C_1$-$C_6$-alkyl, benzyl, phenyl or methylphenyl.

3. An isoindolenine as claimed in claim 1, wherein $R^1$ is methyl or ethyl.

4. An isoindolenine as claimed in claim 1, 2 or 3, wherein $R^2$ is hydrogen, methyl or ethyl.

5. An isoindolenine as claimed in claim 1, 2, 3 or 4, wherein $R^3$ is methyl.

6. An isoindolenine as claimed in claim 1, wherein is morpholino, pyrrolidino or piperidino and

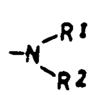

$R^3$ is hydrogen.

7. An isoindolenine as claimed in claim 1, 2, 3, 4, 5 or 6, wherein the benzene ring A is unsubstituted or substituted by t-butyl.

8. A process for preparing an isoindolenine as claimed in claim 1, which comprises condensing a 3-aminophenol of the general formula (II)

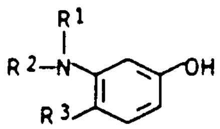

(II)

where $R^1$, $R^2$ and $R^3$ are each as defined in claim 1, with a 3-amino-1-oxoisoindolenine of the general formula (III)

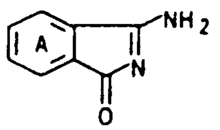

(III)

where the benzene ring A may be substituted by from 1 to 4 chlorines, 1 or 2 $C_1$-$C_4$-alkyls or 1 nitro, in the presence of an acid.

9. A process as claimed in claim 8, wherein the condensation is carried out in an organic solvent in the presence of one equivalent of an acid at from 60 to 140 °C.

10. A process for preparing a 2-(4-amino-2-hydroxybenzoyl)benzoic acid of the general formula (IV)

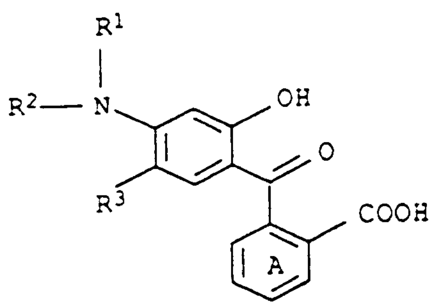

(IV)

where

$R^1$ is hydrogen, unsubstituted or chlorine-, hydroxyl-, phenyl- or cyano-substituted $C_1$-$C_{12}$-alkyl or $C_5$-$C_8$-cycloalkyl or unsubstituted or chlorine- or methyl-substituted phenyl,

$R^2$ is hydrogen or unsubstituted or chlorine-, hydroxyl-, phenyl- or cyano-substituted $C_1$-$C_6$-alkyl, or

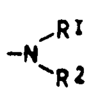

is morpholino, pyrrolidino or piperidino, and

$R^3$ is hydrogen or methyl and the benzene ring A may be substituted by from 1 to 4 chlorines, 1 or 2 $C_1$-$C_4$-alkyls or 1 nitro,

which comprises

a) condensing a 3-aminophenol of the general formula (II)

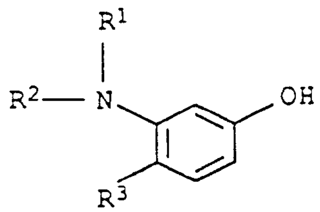

(II)

with a 3-amino-1-oxoisoindolenine of the general formula (III)

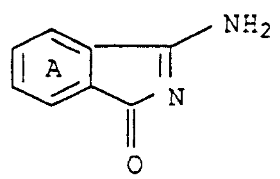

(III)

in the presence of an acid to form a 3-(4-amino-2-hydroxyphenyl)-1-oxoisoindolenine of the general formula (I)

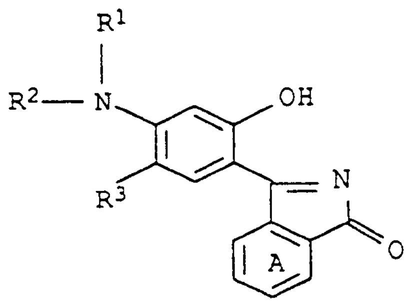

(I)

and
b) subjecting the 3-(4-amino-2-hydroxyphenyl)-1-oxoisoindolenine (I) obtained to hydrolysis.

## Revendications

**1.** Iso-indolénines de formule générale I

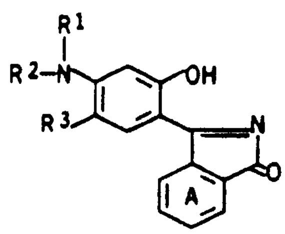

(I),

dans laquelle
$R^1$ représente l'hydrogène, un groupe alkyle en C1-C12, cycloalkyle en C5-C8 éventuellement substitué par le chlore, des groupes hydroxy, phényle ou cyano, ou un groupe phényle éventuellement substitué par le chlore ou des groupes méthyle,
$R^2$ représente l'hydrogène, un groupe alkyle en C1-C6 éventuellement substitué par le chlore, des groupes hydroxy, phényle ou cyano, ou bien

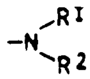

représente un groupe morpholino, pyrrolidino ou pipéridino, et
$R^3$ représente l'hydrogène ou un groupe méthyle,
et le cycle benzénique A est éventuellement substitué par 1 à 4 atomes de chlore, un ou deux groupes alkyle en C1-C4 ou un groupe nitro.

**2.** Iso-indolénines selon la revendication 1, caractérisées en ce que $R^1$ représente un groupe alkyle en C1-C6, benzyle, phényle ou méthylphényle.

**3.** Iso-indolénines selon la revendication 1, caractérisées en ce que $R^1$ représente un groupe méthyle ou éthyle.

**4.** Iso-indolénines selon la revendication 1, 2 ou 3, caractérisées en ce que $R^2$ représente l'hydrogène, un groupe méthyle ou éthyle.

**5.** Iso-indolénines selon les revendications 1, 2, 3 ou 4, caractérisées en ce que $R^3$ représente un groupe méthyle.

**6.** Iso-indolénines selon la revendication 1, caractérisées en ce que

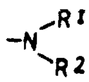

représente un groupe morpholino, pyrrolidino ou pipéridino, et
$R^3$ représente l'hydrogène.

**7.** Iso-indolénines selon les revendications 1, 2, 3, 4, 5 ou 6, caractérisées en ce que le cycle benzénique A est non substitué ou substitué par un groupe tertbutyle.

**8.** Procédé de préparation des iso-indolénines selon la revendication 1, caractérisé en ce que l'on condense en présence d'un acide des 3-aminophénols de formule générale II

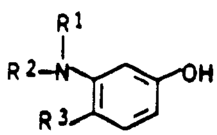

(II),

dans laquelle $R^1$, $R^2$ et $R^3$ ont les significations indiquées dans la revendication 1, avec des 3-amino-1-oxo-iso-indolénines de formule générale III

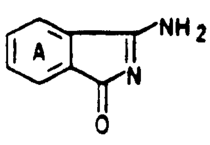

(III),

dans laquelle le cycle benzénique A est éventuellement substitué par 1 à 4 atomes de chlore, 1 ou 2 groupes alkyle en C1-C4 ou un groupe nitro.

**9.** Procédé selon la revendication 8, caractérisé en ce que la condensation est effectuée dans un solvant organique, en présence d'un équivalent d'un acide, à des températures de 60 à 140 °C.

**10.** Procédé de préparation des acides 2-(4-amino-2-hydroxybenzoyl)-benzoiques de formule générale IV

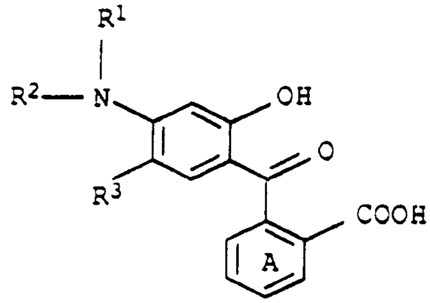

(IV),

dans laquelle
$R^1$ représente l'hydrogène, un groupe alkyle en C1-C12, cycloalkyle en C5-C8 éventuellement substitué par le chlore, des groupes hydroxy, phényle ou cyano, ou un groupe phényle éventuellement substitué par le chlore ou des groupes méthyle,
$R^2$ représente l'hydrogène, un groupe alkyle en C1-C6 éventuellement substitué par le chlore, des groupes hydroxy, phényle ou cyano, ou bien

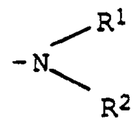

représente un groupe morpholino, pyrrolidino ou pipéridino, et
$R^3$ représente l'hydrogène ou un groupe méthyle,
et le cycle benzénique A est éventuellement substitué par 1 à 4 atomes de chlore, 1 ou 2 groupes alkyle en C1-C4 ou un groupe nitro, caractérisé en ce que :

a) on condense, en présence d'un acide, des 3-aminophénols de formule générale II

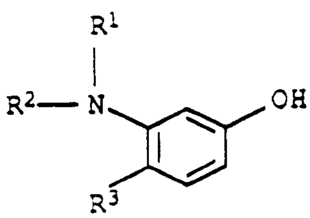

(II)

avec des 3-amino-1-oxo-iso-indolénines de formule générale III

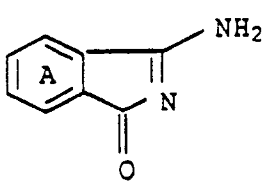

(III)

la condensation donnant des 3-(4-amino-2-hydroxyphényl)-1-oxo-iso-indolénines de formule générale I

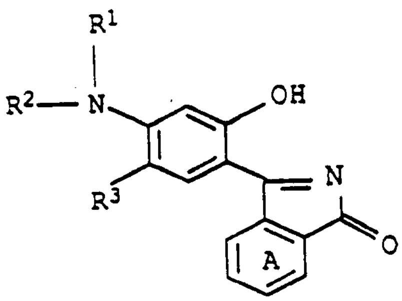

(I),

b) qu'on saponifie.